# EUROPÄISCHE PATENTSCHRIFT

(11) **EP 0 839 789 B1**
(45) Veröffentlichungstag und Bekanntmachung des Hinweises auf die Patenterteilung: **15.12.1999**
(21) Anmeldenummer: 97118404.9
(22) Anmeldetag: 23.10.1997
(51) Int. Cl.: C07C 45/85, C07C 47/544

(54) **Verfahren zur Reinigung von O-Phthaldialdehyd**
Process for the purification of o-phthalic dialdehyde
Procédé pour la purification de dialdéhyde o-phtalique

(30) Priorität: 30.10.1996 AT 189096
(43) Veröffentlichungstag der Anmeldung: 06.05.1998
(73) Patentinhaber: DSM Fine Chemicals Austria GmbH, 4021 Linz (AT)
(72) Erfinder: Giselbrecht, Karl Heinz, Dr., 4061 Pasching (AT); Reiter, Klaus, 4020 Linz (AT); Perndorfer, Eduard, 4050 Traun (AT); Hermanseder, Rudolf, 4624 Pennewang (AT)
(74) Vertreter: Klostermann, Ingrid

(56) Entgegenhaltungen:
- EP-A- 0 147 593
- US-A- 3 492 356
- MIRSADEGHI S ET AL: "1-Methoxyisobenzofuran from base-induced and acid-catalyzed reactions of 1,3-dihydro-1,3-dimethoxyisobenzofuran" J. ORG. CHEM. (JOCEAH,00223263);87; VOL.52 (5); PP.787-92, UNIV. CALIFORNIA;DEP. CHEM.; SANTA BARBARA; 93106; CA; USA (US), XP002052176

## Beschreibung

Phthalaldehyde finden in vielen Gebieten beispielsweise als Zwischenprodukte für die Herstellung von Farbstoffen, optischen Aufhellern oder speziellen Polymeren, Anwendung. Aus diesem Grund sind bereits mehrere Herstellungsvarianten beschrieben. So kann der o-Phthaldialdehyd (OPA) beispielsweise gemäß AT 380.008 durch Ozonolyse von Napthalin mit anschließender Extraktion gewonnen werden. Der Nachteil bei diesem Verfahren ist, daß der sich als Nebenprodukt bildende Ester nur schwer und unzureichend vom OPA abgetrennt werden kann. Weiters stellt OPA eine reaktive Verbindung dar, die thermisch und oxidativ nicht stabil ist.

Um den Aldehyd vor ungewollten Umsetzungen zu schützen, wurde in EP-A1-0522 312 die Möglichkeit beschrieben o-Phthaldialdehydtetraalkylacetale als Depotverbindungen einzusetzen. Diese Depotverbindungen werden gemäß EP-A1-0522 312 durch elektrochemische Oxidation hergestellt.
Phthalaldehydtetraalkylacetale binden jedoch relativ viel Alkohol, wodurch ein großes Lagervolumen bedingt ist.
Aufgabe der vorliegenden Erfindung war es, ein Verfahren zu finden, das die Herstellung von hochreinem o-Phthalaldehyd über eine Depotverbindung, die weniger Alkohol bindet und somit ein geringeres Lagervolumen und eine kürzere Verseifungszeit bedingt und die einen niedrigeren Siedepunkt aufweist, ermöglicht.

Unerwarteterweise konnte diese Aufgabe durch ein Verfahren gelöst werden, bei welchem OPA über einen Reinigungsschritt mit Dialkoxyphthalan als Zwischenprodukt hergestellt wird.

Gegenstand der vorliegenden Erfindung ist demnach ein Verfahren zur Reinigung von o-Phthalaldehyd, das dadurch gekennzeichnet ist, daß eine alkoholische Lösung enthaltend den rohen, verunreinigten o-Phthalaldehyd bei einer Temperatur zwischen 0 und 60°C auf einen pH-Wert zwischen 0 und 3 gestellt, anschließend mit einer Lauge versetzt und das entsprechende Dialkoxyphthalan aus der organischen Phase extrahiert, destillativ gereinigt und bei Bedarf durch Hydrolyse in den reinen o-Phthalaldehyd überführt wird.

Zur Durchführung des erfindungsgemäßen Verfahrens wird zunächst eine Lösung eines durch ein beliebiges Herstellungsverfahren erhaltenen OPA's in einem Alkohol mit 1 bis 4 C-Atomen hergestellt. Alkohole mit 1 bis 4 C-Atomen sind Methanol, Ethanol, Propanol und Butanol. Bevorzugt werden Methanol und Ethanol, besonders bevorzugt Methanol eingesetzt.

Der zu reinigende OPA kann dabei beispielsweise aus Dimethoxybenzol, auf elektrochemischem Weg oder durch Ozonolyse von Naphthalin hergestellt werden. Bevorzugt wird ein durch Ozonolyse, beispielsweise gemäß AT-PS 380.008, hergestellter OPA verwendet. Wird von einem durch Ozonolyse von Naphthalin hergestellten OPA ausgegangen, so liegt dieser, nach erfolgter Ozonolyse, Reduktion der Peroxide und anschließender Abtrennung des Katalysators bereits als alkoholische Lösung vor.
In dieser Lösung sind neben dem verunreinigten OPA noch Naphthalin, Phthalid, Methoxyphthalan, sowie Aldehydsäuren und Aldehydester, wie etwa Glyoxal, Glykolsäureester oder Glyoxylsäureester, oder Bruchstücke davon bzw. deren Natriumsalze enthalten, wobei der Grad der Verunreinigung von der Güte des Hydrierkatalysators abhängig ist und zwischen 5 und 90 % beträgt. Das erfindungsgemäße Reinigungsverfahren ist dabei unabhängig von der Art und dem Gehalt an Verunreinigungen.
Die den verunreinigten OPA enthaltende alkoholische Lösung wird sodann durch Ansäuern auf einen pH-Wert zwischen 0 und 3, bevorzugt zwischen 0,5 und 2, gestellt. Zum Ansäuern eignen sich dabei Mineralsäuren, wie etwa HCl, H₂SO₄, H₃PO₄, organische Säuren, wie etwa Ameisensäure, Essigsäure, p-Toluolsulfon- oder Methylsulfonsäure oder saure Ionentauscher. Bevorzugt werden Mineralsäuren, besonders bevorzugt H₂SO₄ eingesetzt. Die Temperatur bei diesem Schritt beträgt 0 bis 60°C, bevorzugt 15 bis 30°C. Durch das Ansäuern wird der zu reinigende OPA in das entsprechende Dialkoxyphthalan überführt.

Ein sich eventuell bildender Niederschlag an Natriumsulfat wird gegebenenfalls abgetrennt und die verbleibende Lösung des gebildeten Phthalans wird im nächsten Schritt mit einer bis zu 50 %igen wäßrigen Lauge versetzt. Als Lauge eignen sich beispielsweise Natronlauge oder Kalilauge. Bevorzugt wird Natronlauge, eingesetzt.

Anschließend bzw. gleichzeitig wird der als Lösungsmittel verwendete Alkohol abdestilliert.
Der gewählte Druck und Temperatur sind dabei vom eingesetzten Alkohol abhängig. Nach dem Abdestillieren des Alkohols, verbleibt eine das Phthalan enthaltende Salzlösung. Sind in dieser Lösung feste Salze vorhanden, so wird mit Wasser verdünnt, um diese Salze in Lösung zu bringen.

Während die Verunreinigungen wie Ester, Säuren u.s.w. als Natriumsalze in der wäßrige Phase verbleiben, wird das entsprechende Dialkoxyphthalan mit gängigen Extraktionsmitteln wie etwa mit Ethern, beispielsweise mit Diethylether, Diisopropylether, Methyltertiärbutylether, u.s.w. oder mit Essigester, Toluol, u.s.w. aus der organischen Phase extrahiert.

Bevorzugt werden Methyltertiärbutylether, Essigester und Toluol als Extraktionsmittel eingesetzt. Die gewählte Temperatur während des Extraktionsvorganges ist vom verwendeten Extraktionsmittel abhängig und liegt bevorzugt zwischen Raumtemperatur und 80°C.
Das so erhaltene Dialkoxyphthalan kann destillativ gereinigt werden und liegt sodann als farblose Flüssigkeit vor, die bei Raumtemperatur unbegrenzt lagerbar ist.

Um wieder zum OPA zu gelangen, wird das Dialkoxyphthalan entweder sogleich oder bei Bedarf verseift. Dies erfolgt auf üblichem Weg durch Hydrolyse bei einem pH-Wert zwischen 0 und 7, bevorzugt zwischen 0 und 3 mittels Mineralsäuren wie HCl, H₂SO₄, H₃PO₄ oder organischer Säuren wie Essigsäure, Ameisensäure und p-Toluolsulfon- oder Methansulfonsäure.
Die Reaktionstemperatur hängt dabei vom Alkoxyrest des Phthalans ab und liegt bevorzugt zwischen Raumtemperatur und 100°C. Anschließend wird der sich abspaltende Alkohol und gegebenenfalls die Säure unter Vakuum abdestilliert.
Gegebenenfalls kann der OPA noch umkristallisiert werden, beispielsweise aus Diisopropylether, Essigester, Toluol, Methyltertiärbutylether oder Diethylether.
Durch das erfindungsgemäße Verfahren kann hochreiner OPA mit einem Gehalt von bis zu über 99,7 (bestimmt mittels GC) erhalten werden, der keine oxidierten Nebenprodukte aufweist und somit lagerstabiler ist als ein durch ein aus dem Stand der Technik bekanntes Verfahren erhaltener OPA.

### Beispiel 1

Als Ausgangslösung wurde eine methanolische Hydrierlösung aus der Ozonolyseanlage der Fa. DSM Chemie Linz, erhalten durch Ozonolyse von Naphthalin, verwendet. Die Lösung enthielt, nach Abtrennung des Katalysators, folgende Verbindungen

| | (Flächen % GC) |
|---|---|
| Naphthalin | 16,2 |
| Methoxyphthalan | 8,3 |
| o-Phthalaldehyd (OPA) | 69,0 |
| Dimethoxyphthalan | 1,3 |
| Phthalsäureester | 3,9 |
| Acetalester | 0,4 |
| OPA-Tetramethylacetal | 1,0 |

4000 ml Hydrierlösung obiger Zusammensetzung mit einem pH-Wert von 6,05 wurden mit 7 ml H₂SO₄ conc. auf pH 1,35 gestellt.
Anschließend wurde der ausgefallene Niederschlag (21,8 g) an Natriumsulfat abfiltriert und das Filtrat, das den acetalisierten OPA enthält, mit 150 g 20 %iger Natronlauge versetzt. Um den sich abspaltenden Alkohol zu entfernen, wurde die so erhaltene Lösung bei 50°C und 150 mbar eingedampft, sodaß 306 g Rückstand erhalten wurden. Der Rückstand wurde sodann mit 60 g Wasser und 160 g Toluol und nochmals mit 250 g Toluol extrahiert. Die vereinten Toluolphasen wurden anschließend bei 50°C und 20 mbar am Rotavapor eingedampft.
Ausbeute an rohem Dimethoxyphthalan: 129,1 g
wässrige Phase: Ausbeute: 261 g

### Beispiel 2:

Analog Beispiel 1 wurden 4000 ml Hydrierlösung (pH 6,08) mit 7 ml H₂SO₄ conc. auf einen pH-Wert von 1,32 gestellt und über Nacht gerührt. Nach dem Abfiltrieren des ausgefallenen Niederschlages, wurde das Filtrat mit 150 g 40 %iger NaOH versetzt und anschließend zur Entfernung des abgespaltenen Alkohols am Rotavapor eingedampft. Der verbleibende Sumpf wurde mit 200 ml Wasser und 200 ml Toluol versetzt und das Dimethoxyphthalan extrahiert. Dabei wurde zuerst die sich bildende Zwischenphase abfiltriert und die Toluolphase bei 50°C und 20 mbar eingedampft.
Ausbeute: 109,9 g Roh-Dimethoxyphthalan

### Beispiel 3

Die Ausbeuten an Dimethoxyphthalan aus Beispiel 1 und 2 wurden vereinigt und gemeinsam aufgearbeitet.
Gesamtmenge an Dimethoxyphthalan: 239,0 g.
Die Aufarbeitung erfolgte durch eine fraktionierte Vakuumdestillation:

| Fraktion | Ölbad T(°C) | Sumpf T(°C) | Kopf T(°C) | RLV Ab:Rl | Vakuum (mbar) | Auswaage (g) |
|---|---|---|---|---|---|---|
| 1 | 20-97 | 26-88 | bis 22,8 | 1 : 2 | 13 | 9,9 |
| 2 | * | | | | 13 | 7,2 |
| 3 | ** | 125 | 102 | | 13 | 8,2 |
| 4 | 160 | 125 | 84-109,5 | 1 : 2 | 13 | 8,2 |
| 5 | 160 | 125 | 109,5-111,6 | 1 : 2 | 13 | 18,7 |
| 6 | 160-170 | 125-141 | 111,6-117,6 | Ab. | 13 | 124,1 |
| Sumpf | | | | | | 29,0 |
| RLV: Rücklaufverhältnis Ab: Abnahme Rl: Rücklauf *: Destillationskopf mit Naphthalin zugewachsen Destillation unterbrochen und gereinigt. **: Nach Wiederbeginn Kopf erneut zugewachsen. Beim Erwärmen löste sich der Feststoff. | | | | | | |

Fraktion 6 wurde mit 300 ml Essigsäure/Wassergemisch (1:1) versetzt und am Rotavapor bei 50°C und 100 - 20 mbar eingedampft. Ausbeute: 125 g
Der verbleibende Sumpf wurde sodann mit 300 ml Aceton versetzt und wiederum eingedampft. Ausbeute: 123 g
Da noch keine vollständige Entacetalisierung erreicht war, wurde der Rückstand nochmals mit 200 ml Essigsäure/Wassergemisch (1:1) versetzt und wiederum bei 50°C und 20 mbar eingedampft. Ausbeute: 123 g
Dieser Arbeitsschritt wurde mit 200 ml Essigsäure/Wassergemisch und bei 100 mbar und 60°C wiederholt. Ausbeute: 98 g
Da noch etwa 5 % Acetal vorhanden waren, mußten erneut 100 ml Essigsäure/Wassergemisch zugegeben und bei 60°C und 100 mbar eingedampft werden.
Ausbeute 95,8 g an OPA
Der so erhaltene OPA wurde sodann aus 300 ml Diisopropylether (DIPE) unter Zusatz von 1 g Aktivkohle umkristallisiert und im Kühlschrank bei 4°C abgestellt. Anschließend wurde der gebildete Feststoff abgesaugt und mit 50 ml kaltem DIPE nachgewaschen.
Ausbeute: 75,1 g an OPA
Reinheit: 99,96 (Flächen%, GC)

## Patentansprüche

1. Verfahren zur Reinigung von o-Phthalaldehyd dadurch gekennzeichnet, daß eine alkoholische Lösung enthaltend den rohen, verunreinigten o-Phthalaldehyd bei einer Temperatur zwischen 0 und 60°C auf einen pH-Wert zwischen 0 und 3 gestellt, anschließend mit einer Lauge versetzt und das entsprechende Dialkoxyphthalan aus der organischen Phase extrahiert und destillativ gereinigt und bei Bedarf durch Hydrolyse in den gereinigten o-Phthalaldehyd überführt wird.

2. Verfahren nach Anspruch 1, dadurch gekennzeichnet, daß der rohe, verunreinigte o-Phthalaldehyd in einem Alkohol mit 1 bis 4-C-Atomen gelöst ist.

3. Verfahren nach Anspruch 1, dadurch gekennzeichnet, daß die alkoholische Lösung durch Zugabe einer Mineralsäure, einer organischen Säure oder eines sauren Ionentauschers auf einen pH-Wert zwischen O und 3 gestellt wird.

4. Verfahren nach Anspruch 3, dadurch gekennzeichnet, daß als Mineralsäuren HCl, H₂SO₄ oder H₃PO₄ und als organische Säuren Ameisensäure, Essigsäure oder p-Toluol- oder Methansulfonsäure verwendet werden.

5. Verfahren nach Anspruch 1, dadurch gekennzeichnet, daß als Lauge Natron- oder Kalilauge eingesetzt wird.

6. Verfahren nach Anspruch 1, dadurch gekennzeichnet, daß die Hydrolyse bei einem pH-Wert von 0 bis 3 mittels einer Mineralsäure oder organischen Säure durchgeführt wird.

7. Verfahren nach Anspruch 1, dadurch gekennzeichnet, daß das Dialkoxyphthalan mittels Diethylether, Diisopropylether, Methyltertiärbutylether, Essigester oder Toluol aus der organischen Phase extrahiert wird.

8. Verfahren nach Anspruch 1, dadurch gekennzeichnet, daß ein durch ein beliebiges Herstellverfahren erhaltener roher o-Phthalaldehyd als Ausgangsprodukt eingesetzt wird.

9. Verfahren nach Anspruch 1, dadurch gekennzeichnet, daß eine durch Ozonolyse von Naphthalin in Methanol mit anschließender Reduktion der entstandenen Peroxide und Abtrennung des Hydrierkatalysators erhaltene Lösung als alkoholische Lösung die den rohen, verunreinigten o-Phthalaldehyd enthält, verwendet wird.

## Claims

1. Process for the purification of o-phthalaldehyde characterized in that an alcoholic solution containing the crude, unpurified o-phthalaldehyde is adjusted to a pH between 0 and 3 at a temperature between 0 and 60°C, then an alkaline solution is added, and the corresponding dialkoxyphthalan is extracted from the organic phase and is purified by distillation, and, when required, is converted into the purified o-phthalaldehyde by hydrolysis.

2. Process according to Claim 1, characterized in that the crude, unpurified o-phthalaldehyde is dissolved in an alcohol having 1 to 4 carbon atoms.

3. Process according to Claim 1, characterized in that the alcoholic solution is adjusted to a pH between 0 and 3 by addition of a mineral acid, an organic acid or an acidic ion exchanger.

4. Process according to Claim 3, characterized in that the mineral acid used is HCl, H₂SO₄ or H₃PO₄, and the organic acid used is formic acid, acetic acid, p-toluenesulphonic acid or methanesulphonic acid.

5. Process according to Claim 1, characterized in that the alkaline solution used is sodium hydroxide solution or potassium hydroxide solution.

6. Process according to Claim 1, characterized in that the hydrolysis is carried out at a pH of from 0 to 3 using a mineral acid or organic acid.

7. Process according to Claim 1, characterized in that the dialkoxyphthalan is extracted from the organic phase using diethyl ether, diisopropyl ether, methyl tert-butyl ether, ethyl acetate or toluene.

8. Process according to Claim 1, characterized in that a crude o-phthalaldehyde obtained by any desired preparation process is used as the starting material.

9. Process according to Claim 1, characterized in that a solution obtained by ozonolysis of naphthalene in methanol with subsequent reduction of the resultant peroxides and removal of the hydrogenation catalyst is used as the alcoholic solution containing crude, unpurified o-phthalaldehyde.

## Revendications

1. Procédé de purification d'o-phtalaldéhyde, caractérisé en ce qu'on ajuste entre 0 et 3 le pH d'une solution alcoolique contenant l'o-phtalaldéhyde brut contenant des impuretés, à une température comprise entre 0 et 60°C, ensuite, on mélange la solution avec une lessive, on extrait le dialcoxyphtalanne correspondant à partir de la phase organique, on le purifie par distillation et, lorsqu'on en a besoin, on le transforme en l'o-phtalaldéhyde purifié par hydrolyse.

2. Procédé selon la revendication 1, caractérisé en ce qu'on dissout l'o-phtalaldéhyde brut contenant des impuretés dans un alcool ayant de 1 à 4 atomes de carbone.

3. Procédé selon la revendication 1, caractérisé en ce qu'on ajuste entre 0 et 3 le pH de la solution alcoolique par addition d'un acide minéral, d'un acide organique ou d'un échangeur d'ions acide.

4. Procédé selon la revendication 3, caractérisé en ce qu'on utilise HCl, H₂SO₄ ou H₃PO₄ en tant qu'acides minéraux ou de l'acide formique, de l'acide acétique ou de l'acide p-toluènesulfonique ou méthanesulfonique en tant qu'acides organiques.

5. Procédé selon la revendication 1, caractérisé en ce qu'on utilise une lessive de soude ou de la potasse caustique en tant que lessive.

6. Procédé selon la revendication 1, caractérisé en ce qu'on effectue l'hydrolyse à une valeur de pH de 0 à 3 au moyen d'un acide minéral ou d'un acide organique.

7. Procédé selon la revendication 1, caractérisé en ce qu'on extrait le dialcoxyphtalanne de la phase organique au moyen d'éther diéthylique, d'éther diisopropylique, d'éther de méthyle et de tert-butyle, d'acétate d'éthyle ou de toluène.

8. Procédé selon la revendication 1, caractérisé en ce qu'on utilise comme produit de départ un o-phtalaldéhyde brut obtenu au moyen d'un procédé de préparation quelconque.

9. Procédé selon la revendication 1, caractérisé en ce qu'on utilise, comme solution alcoolique contenant l'o-phtalaldéhyde brut contenant des impuretés, une solution obtenue par ozonolyse de naphtalène dans du méthanol, suivie d'une réduction des peroxydes formés et de la séparation du catalyseur d'hydrogénation.
